# EUROPEAN PATENT SPECIFICATION

(11) **EP 1 212 442 B1**
(45) Date of publication and mention of the grant of the patent: **18.05.2005**
(21) Application number: 00963474.2
(22) Date of filing: 14.09.2000
(51) Int. Cl.: C12N 15/82, C07K 14/415, C12N 5/10, A01H 5/00

(54) **ENHANCED PLANT CELL TRANSFORMATION BY ADDITION OF HOST GENES INVOLVED IN T-DNA INTEGRATION**
ERHÖHTE PFLANZENZELLENTRANSFORMATION DURCH HINZUFÜGEN VON WIRTS-SPEZIFISCHEN GENEN, WELCHE AN DER T-DNA INTEGRATION BETEILIGT SIND
TRANSFORMATION AMELIOREE DE CELLULES DE PLANTE PAR ADJONCTION DE GENES HOTES INTERVENANT DANS L'INTEGRATION DE L'ADN-T

(30) Priority: 15.09.1999 US 154158 P
(43) Date of publication of application: 12.06.2002
(73) Proprietor: Purdue Research Foundation, West Lafayette, IN 47907-1063 (US)
(72) Inventor: GELVIN, Stanton, B., West Lafayette, IN 47906 (US); MYSORE, Kirankumar, S., Mysore, Karnataka (IN)
(74) Representative: Grund, Martin, Dr.
(86) International application number: PCT/US2000/025260
(87) International publication number: WO 2001/020012

(56) References cited:
- EP-A- 1 033 405
- WO-A-00/17364
- WO-A-97/12046
- WO-A-99/61619
- DE-C- 4 309 203
- CITOVSKY V ET AL: "NUCLEAR LOCATIZATION OF AGROBACTERIUM VIRE2 PROTEIN IN PLANT CELLS" SCIENCE,US,AMERICAN ASSOCIATION FOR THE ADVANCEMENT OF SCIENCE,, vol. 256, 26 June 1992 (1992-06-26), pages 1802-1805, XP002024324 ISSN: 0036-8075
- REGENSBURG-TUINK A J G ET AL: "Transgenic Nicotiana glauca plants expressing bacterial virulence gene virF are converted into hosts for nopaline strains of Agrobacterium tumefaciens." NATURE (LONDON), vol. 363, no. 6424, 1993, pages 69-71, XP002164891 ISSN: 0028-0836
- NAM J ET AL: "Identification of T-DNA tagged Arabidopsis mutants that are resistant to transformation by Agrobacterium." MOLECULAR AND GENERAL GENETICS, vol. 261, no. 3, April 1999 (1999-04), pages 429-438, XP002164892 ISSN: 0026-8925
- DATABASE EMBL [Online] ACCESSION NO: AB016879, 24 August 1998 (1998-08-24) NAKAMURA Y., ET AL.: "Arabidopsis thaliana genomic DNA, chromosome 5, P1 clone:MRB17." XP002164916
- HUH GYUNG HYE ET AL: "Structural characteristics of two wheat histone H2A genes encoding distinct types of variants and functional differences in their promoter activity." PLANT MOLECULAR BIOLOGY, vol. 33, no. 5, 1997, pages 791-802, XP002164893 ISSN: 0167-4412
- MYSORE KIRANKUMAR S ET AL: "An Arabidopsis histone H2A mutant is deficient in Agrobacterium T-DNA integration." PROCEEDINGS OF THE NATIONAL ACADEMY OF SCIENCES OF THE UNITED STATES, vol. 97, no. 2, 18 January 2000 (2000-01-18), pages 948-953, XP002164894 Jan. 18, 2000 ISSN: 0027-8424 & DATABASE EMBL [Online] ACCESSION NO: AF204967, 20 April 2000 (2000-04-20) MYSORE K.S. ET AL.: "Arabidopsis thaliana histone H2A (rat5) gene, complete cds." & DATABASE EMBL [Online] ACCESSION NO: AF204968, 20 April 2000 (2000-04-20) MYSORE K.S., ET AL.: "Arabidopsis thaliana histone H2A (RAT5) gene, complete cds."
- PRYMAKOWSKA-BOSAK MARTA ET AL: "Histone H1 overexpressed to high level in tobacco affects certain developmental programs but has limited effect on basal cellular functions." PROCEEDINGS OF THE NATIONAL ACADEMY OF SCIENCES OF THE UNITED STATES, vol. 93, no. 19, 1996, pages 10250-10255, XP002164895 1996 ISSN: 0027-8424

## Description

### BACKGROUND

The invention relates enhanced *Agrobacterium* transformation frequencies of plants due to overexpression of the histone H2A gene encoded by the *Arabidopsis RAT5* gene. *Agrobacterium tumefaciens* is a gram negative soil bacterium that has been exploited by plant biologists to introduce foreign DNA into plants. However, there are some limitations on the use of this transforming vector, e.g. difficulties in transforming monocots, and transforming frequencies may be too low to be useful. Although known for this practical application, the actual mechanism of DNA transfer from bacteria to plants is not completely understood.

*Agrobacterium tumefaciens* genetically transforms plant cells by transferring a portion of the bacterial Ti-plasmid, designated the T-DNA, to the plant, and integrating the T-DNA into the plant genome. Little is known about the T-DNA integration process, and no plant genes involved in integration have previously been identified. The DNA that is transferred from *Agrobacterium* to the plant cell is a segment of the Ti, or tumor inducing, plasmid called the T-DNA (transferred DNA). Virulence (*vir*) genes responsible for T-DNA processing and transfer are reported to lie elsewhere on the Ti plasmid. The role of *vir* genes in T-DNA processing, the formation of bacterial channels for export of T-DNA, and the attachment of bacteria to the plant cell are reported (Sheng and Citovsky, 1996; Zupan and Zambryski, 1997). In contrast, little is known about the role of plant factors in T-DNA transfer and integration. The isolation of a putative plant factor has recently been reported. Ballas and Citovsky showed that a plant karyopherin α (AtKAP α) can interact with VirD2 nuclear localization sequences in a yeast two-hybrid interaction system, and is presumably involved in nuclear translocation of the T-complex. Using a similar approach, a tomato type 2C protein phosphatase, DIG3, that can interact with the VirD2 NLS was identified. Unlike AtKAP α, DIG3 plays a negative role in nuclear import. After the T-DNA/T-complex enters the nucleus, it must integrate into the plant chromosome. Plant chromosomal DNA is packaged into nucleosomes consisting primarily of histone proteins. The incoming T-DNA may have to interact with this nucleosome structure during the integration process. However, T-DNA may preferentially integrate into transcribed regions of the genome. These regions are believed to be temporarily free of histones. How exactly T-DNA integration takes place is unknown. Recent reports have implicated involvement of VirD2 protein in the T-DNA integration process. Plant proteins are also likely to be involved in this process (Deng *et al*., 1998; Ballas and Citovsky, 1997; Tao, *et al*.). Other evidence for the involvement of plant factors in T-DNA transfer and integration comes from identification of several ecotypes of *Arabidopsis* that are resistant to *Agrobacterium* transformation.

To identify plant genes involved in *Agrobacterium*-mediated transformation, a T-DNA tagged *Arabidopsis* library was screened for mutants that are resistant to *Agrobacterium* transformation (*rat* mutants). There are several steps in which plant genes are likely involved in the *Agrobacterium*-mediated transformation process. First, plant-encoded factors could be involved in the initial step of bacterial attachment to the plant ceil surface. Mutants and ecotypes that are deficient in bacterial attachment have been identified and genes involved in bacterial attachment are currently being characterized. The next step in which a plant factor(s) could be involved is the transfer of T-strands from the bacteria to plant cells across the plant cell wall and membrane. After T-DNA/T-complex enters the cytoplasm of the plant cell, plant factors are required to transport the T-complex to the nucleus.

WO 9712046 describes a method for achieving stable integration of an exogenous DNA fragment in intact form into the genome of a eukaryotic cell, particularly a plant cell. The method utilizes constructs comprising Vir genes to improve plants transformation frequency by enhancing T-DNA integration.

An *Arabidopsis* T-DNA tagged mutant, *rat5*, was characterized that is deficient in T-DNA integration and is resistant to *Agrobacterium*-mediated root transformation. Both genetic and DNA blot analyses indicated that there are two copies of T-DNA integrated as a tandem repeat at a single locus in *rat5*. No major rearrangements are in the *rat5* plant DNA immediately surrounding the T-DNA insertion site. These data strongly suggest that in *rat5* the T-DNA had inserted into a gene necessary for *Agrobacterium*-mediated transformation. The sequence of the T-DNA left border-plant junction indicated that the T-DNA had inserted into the 3' untranslated region of a histone H2A gene. This insertion is upstream of the consensus polyadenylation signal. By screening an *Arabidopsis* ecotype Ws cDNA library and sequencing 20 different histone H2A cDNA clones, and by performing a computer data base search, at least six different histone H2A genes were shown. These genes encode proteins that are greater than 90% identical at the amino acid sequence level. Thus, the histone H2A genes comprise a small multi-gene family in *Arabidopsis*.

T-DNA integration does not appear to take place by homologous recombination, believed to be the most common method of foreign DNA integration in prokaryotes and lower eukaryotes, because no extensive homology between the T-DNA and target sequences has been found. T-DNA is reported to integrate by illegitimate recombination (Matsumoto *et al*., 1990; Gheysen *et al*., 1991; Mayerhofer *et al*., 1991; Ohba *et al*., 1995). Illegitimate recombination is the predominant mechanism of DNA integration into the genomes of higher plants (Britt, 1996; Offringa *et al*., 1990; Paszkowski *et al*., 1988).

Information on factors affecting *Agrobacterium* transformation frequencies in plants is needed to improve performance of this method.

### SUMMARY OF THE INVENTION

The invention relates to increased *Agrobacterium* transformation frequencies in plants due to addition of at least one gene involved in host T-DNA integration, to the host plant. In an embodiment, addition of at least one histone H2A gene encoded by the *Arabidopsis RAT5* gene enhances transformation frequencies, most likely due to overexpressing of histone as compound to the host's natural expression levels. The gene can be either in transgenic plants or carried by the transforming agent, T-DNA for practice of the invention.

Overexpression of histone genes of the present invention overcomes the poor performance that limits the use of *Agrobacterium* as a transforming vector. Many plants can be transformed transiently by *Agrobacterium* so they express the transforming gene for a period of time, but are not stably transformed because of T-DNA integration problems. Therefore, transgenic plants are not produced. The gene H2A (*RAT5*) plays an important role in illegitimate recombination of T-DNA into the plant genome and the gene's overexpression enhances transformation.

Transient and stable GUS (β-glucuronidase) expression data and the assessment of the amount of T-DNA integrated into the genomes of wild-type and *rat5 Arabidopsis* plants indicated that the *rat5* mutant is deficient in T-DNA integration needed for transformation. Complementing the *rat5* mutation was accomplished by expressing the wild-type *RAT5* histone H2A gene in the mutant plant. Surprisingly, overexpression of *RAT5* in wild-type plants increased *Agrobacterium* transformation efficiency. Furthermore, transient expression of a *RAT5* gene from the incoming T-DNA was sufficient to complement the *rat5* mutant and to increase the transformation efficiency of wild-type *Arabidopsis* plants. The present invention provides methods and compositions to increase stable transformation frequency in plants using direct involvement of a plant histone gene in T-DNA integration.

### BRIEF DESCRIPTION OF THE DRAWINGS

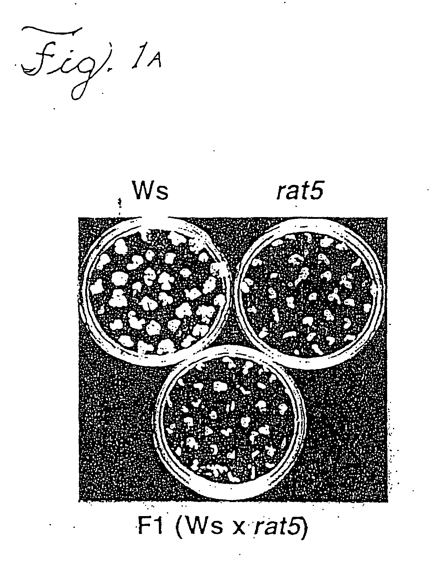

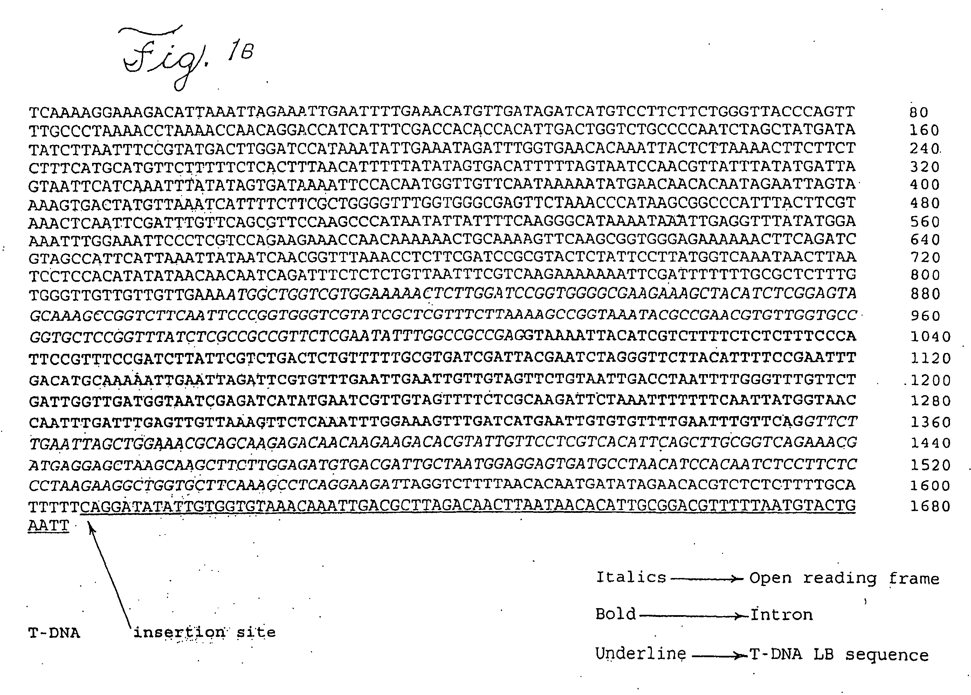

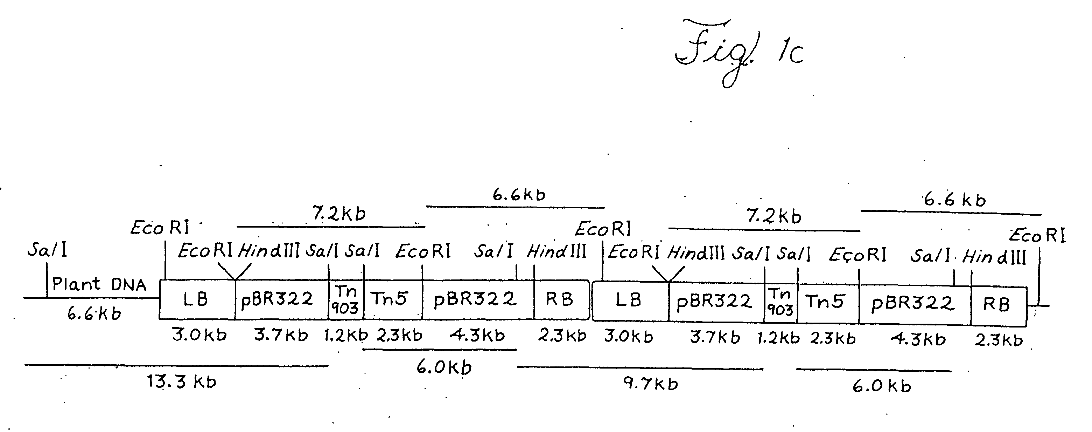

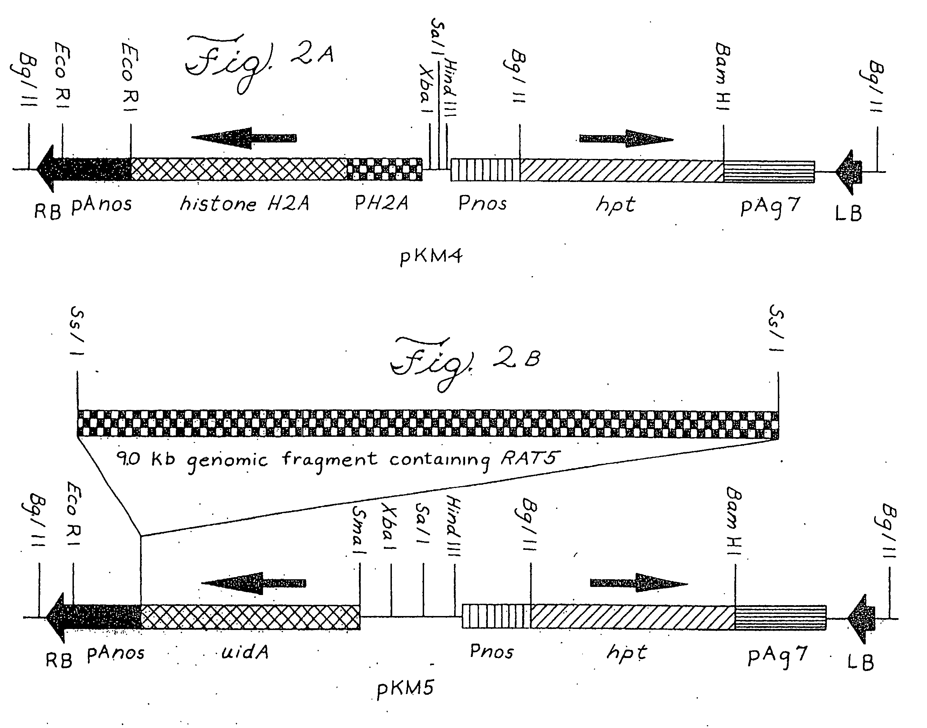

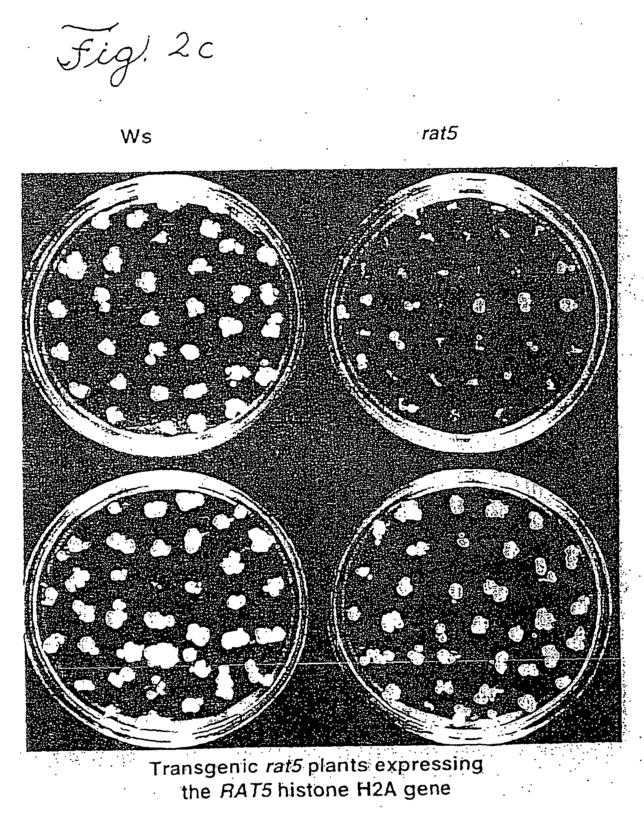

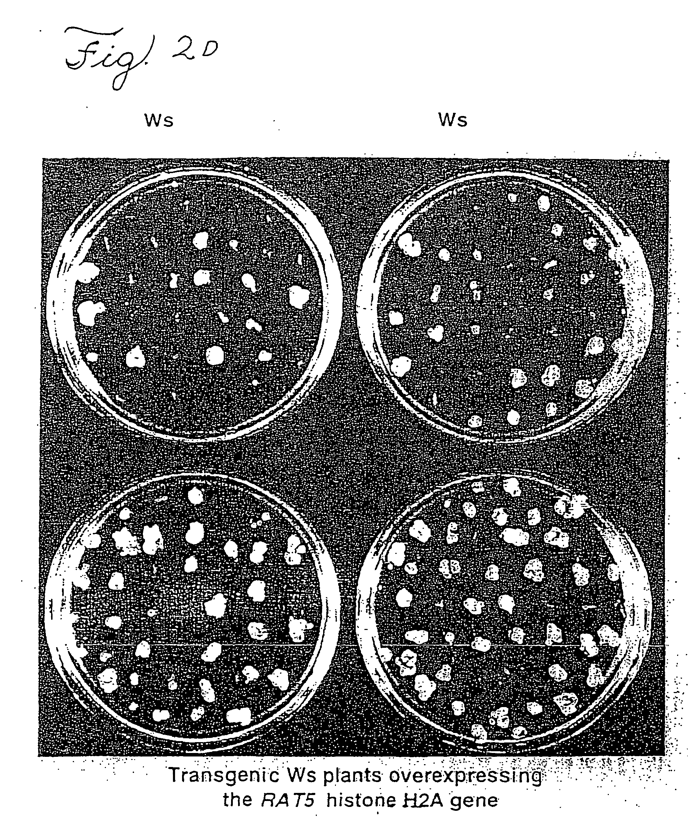

FIG. 1 shows characteristics of the *rat5* mutant: (A) stable transformation of wild-type *Arabidopsis* ecotype Ws, the *rat5* mutant, and the F1 progeny; (B) sequence of the *rat5*/T-DNA junction region; (C) pattern of T-DNA integration in *rat5*: LB, T-DNA left border; RB, T-DNA right border; pBR322, pBR322 sequences containing the β-lactamase gene and ColE1 origin of replication; Tn*903*, kanamycin resistance gene for *E. coli* selection; Tn*5*, kanamycin resistance gene for plant selection.

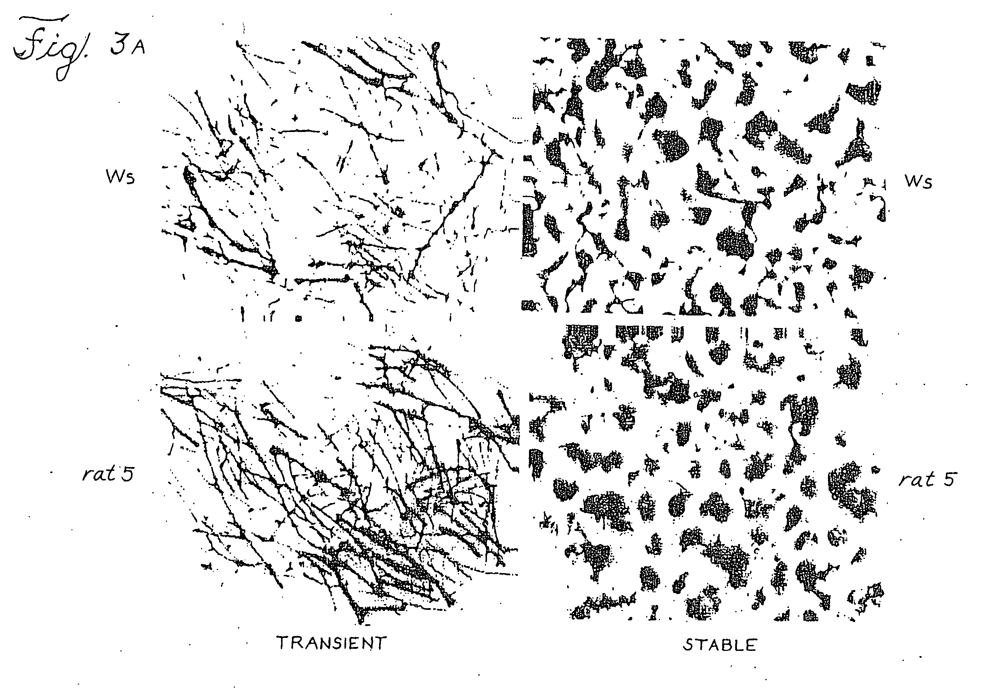

FIG. 2 shows complementation of the *rat5* mutant and overexpression of *RAT5* in wild-type *Arabidopsis* plants; maps of the binary vectors pKM4 (A) and pKM5 (B) RB, T-DNA right border; LB, T-DNA left border; pA*nos*, nopaline synthase polyadenylation signal sequence; *histone H2A*, coding sequence of the *RAT5* histone H2A gene; p*H2A*, promoter sequence of the *RAT5* histone H2A gene; P*nos,* nopaline synthase promoter; *hpt,* hygromycin resistance gene; pAg7, agropine synthase polyadenylation signal sequence; *uidA*, promoterless *gusA* gene; arrows above the *histone H2A, uidA,* and *hpt* genes indicate the direction of transcription; (C) complementation of the *rat5* mutant; (D) tumorigenesis assay of Ws transgenic plants overexpressing the *RAT5* histone H2A gene.

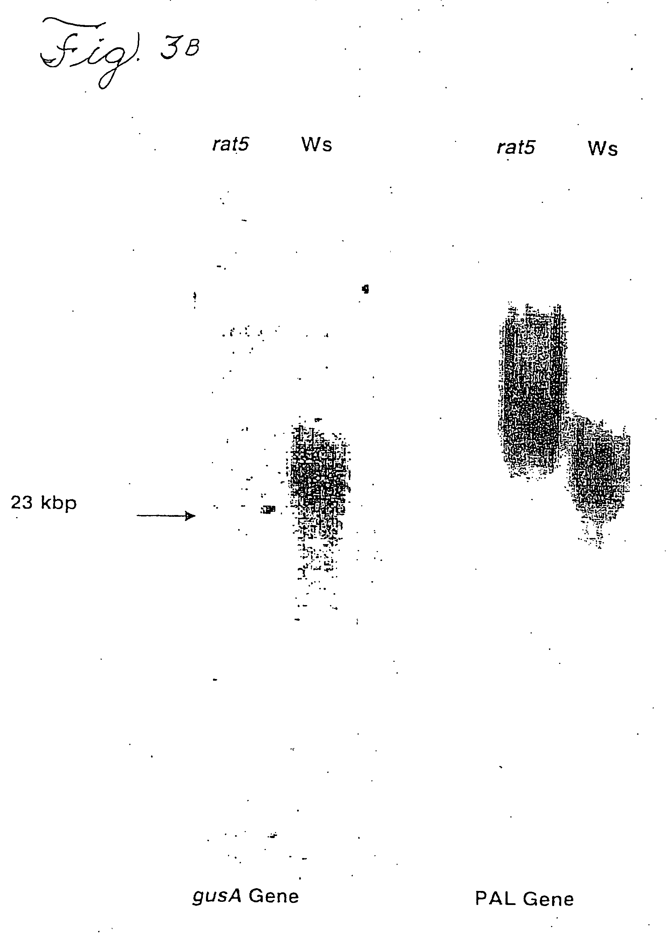

FIG. 3 shows T-DNA integration assays of *rat5* and Ws plants; (A) transient and stable GUS expression in Ws and *rat5*; (B) T-DNA integration in *rat5* and Ws plants.

### DESCRIPTION OF THE INVENTION

Several T-DNA tagged [plants which genes have randomly been disrupted by integration of a T-DNA] mutants of *Arabidopsis* were identified that are recalcitrant to *Agrobacterium* root transformation. These are called *rat* mutants (resistant to *A**grobacterium* transformation). In most of these mutants *Agrobacterium* transformation is blocked at an early step, either during bacterial attachment to the plant cell or prior to T-DNA nuclear import. In some of the mutants, however, the T-DNA integration step is most likely blocked. Because plant factors involved in illegitimate recombination of T-DNA into the plant genome have not previously been identified, the characterization of a T-DNA tagged *Arabidopsis* mutant, *rat5*, that is deficient in T-DNA integration, is an aspect of the present invention.

Characterization of the *rat5* mutant. *rat5*, an *Arabidopsis* T-DNA tagged mutant, was previously identified as resistant to *Agrobacterium* root transformation. An *in vitro* root inoculation assay was performed using the wild-type *Agrobacterium* strain A208 (At 10). After one month, the percentage of root bundles that formed tumors was calculated. Greater than 90% of the root bundles of the wild-type plants (ecotype Ws) formed large green teratomas. In contrast, fewer than 10% of the root bundles from the *rat5* plants responded to infection, forming small yellow calli (FIG. 1A). A homozygous *rat5* plant (pollen donor) was crossed to a wild-type plant (egg donor) and the resulting F1 progeny tested for susceptibility to *Agrobacterium* transformation. This analysis indicated that *rat5* is a dominant mutation (7; FIG. 1A). Further analysis of F2 progeny indicated that kanamycin resistance segregated 3:1, indicating that a single locus had been disrupted by the mutagenizing T-DNA. Kanamycin resistance co-segregated with the *rat5* phenotype, indicating that a gene involved in *Agrobacterium* transformation had most likely been mutated by the T-DNA insertion.

Recovery of a T-DNA-plant junction from *rat5*. The T-DNA integration pattern in the *rat5* mutant was determined by DNA blot analyses. The results indicated that there are only two copies of the mutagenizing T-DNA integrated into the genome of the *rat5* mutant. Further analysis indicated that these two T-DNA copies are present as a direct tandem repeat, as shown in FIG. 1C.

A left border (LB) T-DNA-plant junction was recovered from *rat5* using a plasmid rescue technique (see Materials and Methods) and a restriction endonuclease map of this T-DNA-plant junction was constructed. An approximately 1.7 kbp *Eco*RI fragment that contains both plant and LB DNA was subcloned into pBluescript and subsequently sequenced at the Purdue University sequencing center. The sequence of this fragment is shown in FIG. 1B. DNA sequence analysis of this junction region indicated that the T-DNA had inserted into the 3' untranslated region (UTR) of a histone H2A gene (FIG. 1B). The histone H2A genes of *Arabidopsis* were further characterized by isolating and sequencing numerous cDNA and genomic clones. Six different gene variants of histone H2A were identified, indicating that the histone H2A genes of *Arabidopsis* comprise a small multi-gene family. In a lambda genomic DNA library a clone was identified containing the wild-type histone H2A gene corresponding to *RAT5*. DNA sequence analysis of this genomic clone indicated that in *rat5* the T-DNA had inserted upstream of the consensus polyadenylation signal (AATAA). DNA blot analysis of Ws and *rat5* DNA indicated that the T-DNA insertion in *rat5* did not cause any major rearrangements in the plant DNA immediately around the site of insertion. Disruption of the 3' UTR of the *RAT5* histone H2A gene is likely the sole cause for the *rat* phenotype in the *rat5* mutant.

FIG. 1 shows characterization of the *rat5* mutant. (A) Stable transformation of wild-type *Arabidopsis* ecotype Ws, the *rat5* mutant, and the F1 progeny. Sterile root segments were infected with *A. tumefaciens* A208. Two days after cocultivation, the roots were transferred to MS medium lacking phytohormones and containing timentin as an antibiotic. Tumors were scored after four weeks. (B) Sequence of the *rat5*/T-DNA junction region. (C) Pattern of T-DNA integration in *rat5*. LB, T-DNA left border; RB, T-DNA right border; pBR322, pBR322 sequences containing the β-lactamase gene and ColE1 origin of replication; Tn*903*, kanamycin resistance gene for *E. coli* selection; Tn5, kanamycin resistance gene for plant selection. Five µg of genomic DNA from the *rat5* mutant was digested with either *Eco*RI or *Sal*I and was blotted onto a nylon membrane. An *Eco*RI*-Sal*I fragment of pBR322 was used as the hybridization probe. Restriction fragment sizes shown above the T-DNA were detected by *Eco*RI digestion and the sizes shown below the T-DNA were detected by *Sal*I digestion.

Complementation of the *rat5* mutant with a wild-type histone H2A gene (*RAT5*). Two different constructions were made to perform a complementation analysis of the *rat5* mutant. First, a nopaline synthase terminator (3' NOS) was fused to the 3' region of the 1.7 kbp junction fragment (the sequence of this 1.7 kbp fragment is shown in FIG. 1B). This construction contains the *RAT5* histone H2A gene with its own promoter and a 3' NOS. This fragment (*RAT5* plus 3' NOS) was cloned into the binary vector pGTV-HPT of beaker containing a hygromycin resistance gene between the left and the right T-DNA borders, resulting in the binary vector pKM4 (FIG. 2A). For the second construction, a 9.0 kbp *Sac*I genomic fragment of wild-type Ws DNA containing a histone H2A gene (*RAT5*) plus at least 2.0 kbp sequences upstream and downstream of *RAT5* was cloned into the binary vector pGTV-HPT, resulting in the binary vector pKM5 (FIG. 2B). pKM4 and pKM5 were transferred separately into the non-tumorigenic *Agrobacterium* strain GV3101, resulting in strains *A. tumefaciens* At1012 and At1062, respectively.

Both strains At1012 and At1062 were separately used to transform *rat5* plants using a germ-line transformation method (Bent et al., 1998) and transgenic *rat5* plants were selected for resistance to hygromicin (20 µg/ml). Several transgenic plants (T1) were obtained. These transgenic plants were allowed to self fertilize and T1 seeds were collected. Six transgenic lines obtained by transformation with At1012 (the wild-type histone H2A with 3' NOS) were randomly selected and their seeds were germinated in the presence of hygromycin. Tumorigenesis assays were performed as described in Nam et al. (1999) using *A. tumefaciens* At 10 and a sterile root inoculation protocol, on at least five different plants from each of the six transgenic lines. The results indicated that in five of the six transgenic *rat5* lines tested, the tumorigenesis-susceptibility phenotype was recovered (FIG. 2C; Table 1). Teratomas incited on the roots of these plants appeared similar to tumors generated on a wild-type plant. One of the transgenic plants tested did not recover the tumorigenesis-susceptibility phenotype, probably because of an inactive transgene. Transgenic T1 plants of *rat5* obtained by transformation with At1062 (containing a genomic encoding *RAT5* from the wild-type plant) were also tested for restoration of the tumorigenesis-susceptibility phenotype. Some of these plants were also able to recover the tumorigenesis-susceptibility phenotype, indicating complementation of the *rat5* mutation. Hygromycin-resistant transgenic plants generated by transforming the *rat5* mutant with pGPTV-HPT alone did not form tumors upon infection with *A. tumefaciens* A208.

To confirm the genetic basis of the complementation experiment, a co-segregation analysis was performed on one of the *rat5* transgenic lines (*rat5* At1012-6) obtained by transformation of the *rat5* mutant with *A. tumefaciens* At1012. To examine the co-segregation of the complementing T-DNA containing the wild-type *RAT5* gene with the tumorigenesis-susceptibility phenotype, seeds from a T2 plant homozygous for the *rat5* mutation but heterozygous for hygromycin resistance were germinated and grown on B5 medium without selection. Roots of these plants were subsequently tested for hygromycin-resistance and susceptibility to crown gall tumorigenesis. All plants that were sensitive to hygromycin were also resistant to tumor formation in a manner similar to that of the *rat5* mutant. Of the 25 hygromycin-resistant plants, at least 8 were susceptible to tumorigenesis. However, 17 hygromycin-resistant plants remained recalcitrant to *Agrobacterium-*mediated transformation. It is likely that these plants are heterozygous with respect to the complementing *RAT5* gene and did not express this gene to a level high enough to restore susceptibility to tumorigenesis. This possibility corresponds to the finding that the *rat5* mutation is dominant, and that therefore one active copy of *RAT5* is not sufficient to permit *Agrobacterium*-mediated transformation. Taken together, the molecular and genetic data strongly indicate that in the *rat5* mutant disruption of a histone H2A gene is responsible for the tumorigenesis-deficiency (*rat*) phenotype.

Overexpression of a histone H2A (*RAT5*) gene in wild-type plants improves the efficiency of *Agrobacterium* transformation. To determine further whether the *RAT5* gene plays a direct role in *Agrobacterium-*mediated transformation, *A. tumefaciens* At1012 was used to generate several transgenic *Arabidopsis* plants (ecotype Ws) containing additional copies of the *RAT5* histone H2A gene. These transgenic plants were allowed to self-pollinate, T1 seeds were collected, and T2 plants were germinated in the presence of hygromycin. Tumorigenesis assays were performed as described herein at least five plants from each of four different transgenic lines. Because ecotype Ws normally is highly susceptible to *Agrobacterium* transformation, the tumorigenesis assay was altered to detect any subtle differences between the transformation-susceptible wild-type plant and transgenic wild-type plants overexpressing *RAT5*. These alterations included inoculation of root segments with a 100-fold lower concentration (2x10⁷ cfu/ml) of bacteria than that normally used (2x10⁹ cfu/ml), and spreading individual root segments rather than bundles of root segments on MS medium to observe tumor production. The results, shown in Table 1 and FIG. 2D, indicate that transgenic plants overexpressing *RAT5* are approximately twice as susceptible to root transformation as are wild-type Ws plants. These data indicate that the *RAT5* histone H2A gene plays a direct role in T-DNA transformation, and that overexpression of *RAT5* can increase susceptibility to transformation.

Transient expression of histone H2A is sufficient to permit transformation of *rat5* and to increase the transformation efficiency of wild-type Ws plants. Expression of the *RAT5* histone H2A gene from the incoming T-DNA complement the *rat5* mutant. Although transformation of this mutant with an *Agrobacterium* strain harboring pGPTV-HYG (lacking a histone H2A gene) resulted in only a few, slow-growing calli on hygromycin selection medium, *Agrobacterium* strains harboring pKM4 or pKM5 incited rapidly growing hygromycin-resistant calli on 60±21% and 54±22% of the *rat5* root segment bundles, respectively. In addition, when wild-type plants were infected (at low bacterial density) with a tumorigenic *Agrobacterium* strain (A208) harboring pKM4, 78±8% of the root segments developed tumors, compared to 36±9% of the root segments infected with a tumorigenic bacterial strain harboring pGPTV-HYG. These transformation experiments indicate that *Agrobacterium* strains containing the binary vectors pKM4 or pKM5 are able to transform *rat5* mutant plants at relatively high efficiency, and on wild-type plants are two-fold more tumorigenic, and are better able to incite hygromycin-resistant calli, than are *Agrobacterium* strains containing the "empty" binary vector pGPTV-HYG. Transiently produced histone H2A may improve the stable transformation efficiency of plants by *Agrobacterium.*

The *rat5* mutant is deficient in T-DNA integration. *Agrobacterium*-mediated transformation of the *Arabidopsis rat5* mutant results in a high efficiency of transient transformation but a low efficiency of stable transformation, as determined by the expression of a *gusA* gene encoded by the T-DNA. This result suggested that *rat5* is most likely deficient in T-DNA integration. To test this hypothesis directly root segments from Ws and *rat5* plants were inoculated with *A. tumefaciens* GV3101 harboring the T-DNA binary vector pBISN1. pBISN1 contains a *gusA*-intron gene under the control of a "super-promoter" (Ni et al., 1995; Narasimhulu et al., 1996). Two days after cocultivation, the root segments were transferred to callus inducing medium containing timentin (100 µg/ml) to kill the bacteria. Three days after infection, a few segments were stained for GUS activity using the chromogenic dye X-gluc. Both the wild-type and the *rat5* mutant showed high levels of GUS expression (approximately 90% of the root segments stained blue; FIG. 3A). The remaining root segments were allowed to form calli on callus inducing medium containing timentin to kill *Agrobacterium,* but lacking any antibiotic for selection of plant transformation. After four weeks numerous calli derived from at least five different Ws and *rat5* plants were stained with X-gluc. Of the Ws calli sampled, 92±12% showed large blue staining areas, whereas only 26±10% of the *rat5* calli showed GUS activity, and most of these blue staining regions were small (FIG. 3A). These data indicate that although the *rat5* mutant can transiently express the *gusA* gene at high levels, it fails to stabilize *gusA* expression.

Suspension cell lines were generated from these Ws and *rat5* calli and after an additional month the amount of T-DNA was assayed (using as a hybridization probe the *gusA*-intron gene located within the T-DNA of pBISN1) integrated into high molecular weight plant DNA from Ws and *rat5* calli (Nam et al., 1997; Mysore et al., 1998). FIG. 3B shows that although T-DNA integrated into the genome of wild-type Ws plants was easily detectable, T-DNA integrated into the *rat5* genome was not. These data directly demonstrate that *rat5* is deficient in T-DNA integration. To demonstrate equal loading of plant DNA in each of the lanes, the *gusA* probe was stripped from the blot and rehybridized the blot with an *Arabidopsis* phenylalanine ammonia-lyase (PAL) gene probe.

FIG. 2 shows complementation of the *rat5* mutant and overexpression of *RAT5* in wild-type *Arabidopsis* plants. Maps of the binary vectors pKM4 (**A**) and pKM5 (**B**). RB, T-DNA right border; LB, T-DNA left border; pA*nos*, nopaline synthase polyadenylation signal sequence; *histone H2A*, coding sequence of the *RAT5* histone H2A gene; pH2A, promoter sequence of the *RAT5* histone H2A gene; Pnos, nopaline synthase promoter; *hpt,* hygromycin resistance gene; pAg7, agropine synthase polyadenylation signal sequence; *uidA*, promoterless *gusA* gene. Arrows above the *histone H2A, uidA,* and *hpt* genes indicate the direction of transcription. (**C**) Complementation of the *rat5* mutant. *rat5* mutant plants were transformed with an *Agrobacterium* strain containing the binary vector pKM4 (At1012). Hygromycin-resistant transgenic plants were obtained and were self-pollinated to obtain T2 plants. Sterile root segments of T2 plants expressing *RAT5*, wild-type Ws plants, and *rat5* mutant plants were infected with the tumorigenic strain *A. tumefaciens* A208. Two days after cocultivation, the roots were moved to MS medium lacking phytohormones and containing timentin. Tumors were scored after four weeks. (**D**) Tumorigenesis assay of Ws transgenic plants overexpressing the *RAT5* histone H2A gene. Ws plants were transformed with *A. tumefaciens* At1012 containing the binary vector pKM4. Hygromycin-resistant transgenic plants were obtained and were self-pollinated to obtain T2 plants. Sterile root segments of T2 plants overexpressing *RAT5* and wild-type Ws plants were infected at low bacterial density with *A. tumefaciens* A208. After two days cocultivation, the roots were moved to MS medium lacking phytohormones and containing timentin. Tumors were scored after four weeks.

Teratomas incited on the roots of these plants appeared similar to tumors generated on a wild-type plant. One of the transgenic plants tested did not recover the tumorigenesis-susceptibility phenotype, probably because of an inactive transgene. Transgenic T1 plants of *rat5* obtained by transformation with At1062 (containing a genomic encoding *RAT5* from the wild-type plant) were also tested for restoration of the tumorigenesis-susceptibility phenotype. Some of these plants were also able to recover the tumorigenesis-susceptibility phenotype, indicating complementation of the *rat5* mutation. Hygromycin-resistant transgenic plants generated by transforming the *rat5* mutant with pGPTV-HPT alone did not form tumors upon infection with *A. tumefaciens* A208.

FIG. 3 shows T-DNA integration assays of *rat5* and Ws plants; (A) transient and stable GUS expression in Ws and *rat5*; Sterile root segments of Ws and *rat5* plants were infected with the non-tumorigenic *Agrobacterium* strain GV3101 containing the binary vector pBISN1. Two days after cocultivation, the roots were transferred to callus inducing medium (CIM) containing timentin. Three days after infection, half of the segments were stained with X-gluc to determine the efficiency of transient GUS expression. The other group of segments was allowed to form calli on CIM. After four weeks these calli were stained with X-gluc to determine the efficiency of stable GUS expression. (B) T-DNA integration in *rat5* and Ws plants. Suspension cells were derived from the calli generated from Ws and *rat5* root segments infected with the non-tumorigenic *Agrobacterium* strain GV3101 containing the binary vector pBISN1. The suspension cell lines were grown for three weeks (without selection for transformation) in the presence of timentin or cefotaxime to kill *Agrobacterium*. Genomic DNA was isolated from these cells, subjected to electrophoresis through a 0.6% agarose gel, blotted onto a nylon membrane, and hybridized with a *gusA* gene probe. After autoradiography, the membrane was stripped and rehybridized with a phenylalanine ammonia-lyase (PAL) gene probe to determine equal loading of DNA in each lane.

### MATERIALS AND METHODS

**Nucleic acid manipulation**. Total plant genomic DNA was isolated according to the method of Dellaporta et al. (1983). Restriction endonuclease digestions, agarose gel electrophoresis, plasmid isolation, and DNA blot analysis were conducted as described (Sambrook et al., 1982).

**Plasmid Rescue.** Genomic DNA (5 µg) of *rat5* was digested to completion with *Sal*I. The digested DNA was extracted with phenol/chloroform and precipitated with ethanol. The DNA was self-ligated in a final volume of 500 µl in 1 x ligation buffer (Promega) with 3 units of T4 DNA ligase at 16°C for 16 hr. The ligation mixture was precipitated with ethanol, transformed into electrocompetent *E. coli* DH10B cells (*mcr*BC-; Life Technologies, Inc., Gaithersburg, MD) by electroporation (25 µF, 200 Ω, and 2.5 kV) and plated on LB medium containing ampicillin (100 µg/ml). Ampicillin-resistant colonies were lifted onto a nylon membrane, the bacteria were lysed, and DNA was denatured *in situ* (Sambrook *et al*., 1982). A radiolabeled left border (LB) sequence (3.0 kbp *Eco*RI fragment of pE1461) was used as a hybridization probe to identify a plasmid containing the LB. Positive colonies were picked and plasmid DNA was isolated. By restriction fragment analysis a plasmid containing both the LB and plant junction DNA was identified. The plant junction fragment was confirmed by hybridizing the junction fragment to wild-type plant DNA. A restriction map of this plasmid, containing the LB-plant junction DNA, was made. A 1.7 kbp *Eco*RI fragment that contained plant DNA plus 75 base pairs of LB sequence was subcloned into pBluescript, resulting in pE1509. This fragment was subsequently sequenced at the Purdue University sequencing center.

### Growth of Agrobacterium and in vitro root inoculation of Arabidopsis thaliana These were performed as described previously by Nam et al. (1997).

**Plant Growth Conditions** Seeds of various *Arabidopsis thaliana* ecotypes were obtained from S. Leisner and E. Ashworth (originally from the Arabidopsis Stock Centre, Nottingham, UK, and the *Arabidopsis* Biological Resource Center, Ohio State University, Columbus, respectively). Seeds were surface sterilized with a solution composed of 50% commercial bleach and 0.1% SDS for 10 min and then rinsed five times with sterile distilled water. The seeds were germinated in Petri dishes containing Gamborg's B5 medium (GIBCO) solidified with 0.75% bactoagar (Difco). The plates were incubated initially at 4°C for 2 days and the fro 7 days under a 16-hr-lights/8-hr-dark photoperiod at 25 °C. Seedlings were individually transferred into baby food jars containing solidified B5 medium and grown for 7 to 10 days for root culture. Alternatively, the seedlings were transferred into soil for bolt inoculation.

**Growth of** ***Agrobacterium tumefaciens*** All Agrobacterium strains were grown in YEP medium (Lichtenstein and Draper, 1986) supplemented with the appropriate antibiotics (rifampicin, 10µg/mL; kanamycin, 100 µg/mL) at 30°C. Overnight bacterial cultures were washed with 0.9% NaCl and resuspended in 0.9% NaCl a 2 x 10⁹ colony-forming units per mL for *in vitro* root inoculation or at 2 x 10¹¹ colony-forming units per mL for bolt inoculation.

***In Vitro*** **Root Inoculation and Transformation Assays** Roots grown on the agar surface were excised, cut into small segments (-0.5 cm) in a small amount of sterile water, and blotted onto sterile filter paper to remove excess water. For some experiments, excised roots were preincubated on callus-inducing medium (CIM;4.32 g/L Murashige and Skoog [MS] minimal salts [GIBCO], 0.5 g/L Mes, pH 5.7, 1mL/L vitamin stock solution [0.5mg/mL nicotinic acid, 0.5 mg/mL pyridoxine, and 0.5 mg/mL thyamine-HCl], 100 mg/L myoinositol, 20 g/L glucose, 0.5 mg/L 2,4-dichlorophenoxyacetic acid, 0.3 mg/L kinetin, 5mg/L indoleacetic acid, and 0.75% bactoagar) for 1 day before cutting them into segments. Dried bundles of root segments were transferred to MS basal medium (4.32 g/L MS minimal salts, 0.5 g/L Mes, pH 5.7, 1 mL/L vitamin stock solution, 100 mg/L myoinositol, 10g/L sucrose and 0.75% bactoagar), and 2 or 3 drops of bacterial suspension were placed on them. After 10 min, most of the bacterial solution was removed, and the bacteria and root segments were cocultivated at 25°C for 2 days.

For transient transformation assays, the root bundles were infected with *Agrobacterium* strain GV3101 was used (Koncz and Schell, 1986) containing the binary vector pBISN1 (Narasimhulu *et al*., 1996). After various periods of time, the roots were rinsed with water, blotted on filter paper, and stained with X-gluc staining solution (50 mM NaH₂HPO₄, 10 mM Na₂ · EDTA, 300 mM mannitol, and 2 mM X-gluc, pH 7.0) for 1 day at 37°C. For quantitative measurements of β-glucuronidase (GUS) activity, the roots were ground in a microcentrifuge tube containing GUS extraction buffer (50 mM Na₂HPO₄, 5 mM DTT, 1 mM Na₂ EDTA, 0.1% sarcosyl, and 0.1% Triton X-100, pH 7.0), and GUS specific activity was measured according to Jefferson *et al*. (1987).

To quantitate tumorigenesis, root bundles were infected with wild-type *Agrobacterium* strains. After 2 days, the root bundles were rubbed on the agar surface to remove excess bacteria and then washed with sterile water containing timentin (100µg/mL). Individual root segments (initial assay) or small root bundles (5 to 10 root segments; modified assay) were transferred onto MS basal medium lacking hormones but containing timentin (100µg/mL) and incubated for 4 weeks.

For transformation of root segments to kanamycin resistance, root bundles were inoculated with *Agrobacterium* strain GV3101 containing pBISN1. After 2 days, small root bundles (or individual root segments) were transferred onto CIM containing timentin (100µg/mL) and kanamycin (50µg/mL). Kanamycin-resistant calli were scored after 4 weeks of incubation at 25°C.

To determine stable GUS expression, roots were inoculated as given above and the root segments were transferred after 2 days to CIM containing timentin (100µg/mL) without any selection. After 4 weeks, GUS activity was assayed either by staining with X-gluc or by measuring GUS specific activity by using a 4-methylumbelliferyl β-D galactoside (MUG) fluorometric assay, as described above.

To determine the kinetics of GUS expression, root bundles were infected, the root segments were transferred after 2 days to CIM containing timentin (100µg/mL), and calli were grown on CIM without selection. Root bundles were assayed at various times, using a MUG fluorometric assay as described above, to measure GUS specific activity.

**Construction of the binary vectors pKM4 and pKM5.** The plasmid pE1509 containing the 1.7 kbp junction fragment cloned into pBluescript was digested with *Eco*RI to release the junction fragment. The 5' overhanging ends were filled in using the Klenow fragment of DNA polymerase I and deoxynucleotide triphosphates. The T-DNA binary vector (pE1011) pGTV-HPT (Becker et al., 1992) was digested with the enzymes *Sac*I and *Sma*I, releasing the promoterless *gusA* gene from pGTV-HPT. The 3' overhanging sequence of the larger fragment containing the origin of replication and the hygromycin resistance gene (*hpt*) were removed using the 3'-5' exonuclease activity of Klenow DNA polymerase, and the resulting 1.7 kbp blunt end fragment was ligated to the blunt ends of the binary vector. A binary vector plasmid containing the 1.7 kbp fragment in the correct orientation (pAnos downstream of the histone H2A gene) was selected and named pKM4 (strain E1547).

An approximately 9.0 kbp wild-type genomic *SacI* fragment containing the histone H2A gene (*RAT5*) from a lambda genomic clone was cloned into the *Sac*I site of the plasmid pBluescript. This 9.0 kbp *Sac*I fragment was subsequently released from pBluescript by digestion with *Sac*I and was cloned into the *Sac*I site of the binary vector pGTV-HPT, resulting in the plasmid pKM5 (strain E1596). Both pKM4 and pKM5 were separately transferred by triparental mating (Ditta et al., 1980) into the non-tumorigenic *Agrobacterium* strain GV3101, resulting in the strains *A. tumefaciens* At1012 and At1062, respectively.

**Germ-line transformation of** ***Arabidopsis***. Germ-line transformations were performed as described in (Bent and Clough, 1998). Transgenic plants were selected on B5 medium containing hygromycin (20 µg/ml).

### DOCUMENTS CITED

Ballas, N. & Citovsky, V. Nuclear localization signal binding protein from *Arabidopsis* mediates nuclear import of *Agrobacterium* VirD2 protein. *Proc. Natl. Acad. Sci*. USA 94, 10723-14728 (1997).
Bent, A. F. & Clough, S. J. in *Plant Molecular Biology Manual*, (eds Gelvin, S. B. & Verma, D. P. S.) vol. 3, pp. B7/1-14 (Kluwer Academic Publishers, Netherlands, 1998).
Britt, A. B. DNA damage and repair in plants. *Annu. Rev. Plant Physiol. Plant Mol. Biol*. 47, 75-100 (1996).
Dellaporta, S.L., Wood, J., & Hicks, J.B. *Plant Mol. Biol. Rep.* **1**, 19-22 (1983).
Deng W. *et al. Agrobacterium* VirD2 protein interacts with plant host cyclophilins. *Proc. Natl. Acad. Sci*. USA 95,7040-7045 (1998).
Ditta, G., Stanfield, S., Corbin, D., & Helinski, D.R. *Proc. Natl. Acad. Sci USA* 77, 7347-7351 (1980).
Gheysen, G., Villarroel, R. & Van Montagu, M. Illegitimate recombination in plants: A model for T-DNA integration. *Genes Dev.* 5,287-297 (1991).
Jefferson, R.A.m Kavanagh, T.A., Bevan, M. W. GUS fusions: Betaglucuronidase as a sensitive and versitile gene fusion marker in higher plants. *EMBO J*. **6**, 391-3907 (1987).
Koncz, C. and Schell, J. *Mol. Gen. Genet.* 204, 383-396 (1986).
Lichtenstein , C. and Draper, J. Genetic engeneering of plants. In *Glover, D*.*M*. (ed.) *DNA Cloning: A Practical Approach,* vol. 2, pp. 67-119 (IRL Press, Oxford, 1986).
Matsumoto, S., Ito, Y., Hosoi, T., Takahashi, Y. & Machida, Y. Integration of *Agrobacterium* T-DNA into tobacco chromosome: Possible involvement of DNA homology between T-DNA and plant DNA. *Mol. Gen. Genet.* 224, 309-316 (1990).
Mysore, K. S., Yi, H. C. & Gelvin, S. B. Molecular cloning, characterization and mapping of histone H2A genes in *Arabidopsis.* In preparation.
Mysore, K. S. *et al*. Role of the *Agrobacterium tumefaciens* VirD2 protein in T-DNA transfer and integration. *Mol. Plant-Microbe Interact.* 11, 668-683 (1998).
Nam J. *et al*. Identification of T-DNA tagged *Arabidopsis* mutants that are resistant to transformation by *Agrobacterium. Mol. Gen. Genet.* 261, 429-438 (1999).
Nam, J., Matthysse, A. G. & Gelvin, S. B. Differences in susceptibility of *Arabidopsis* ecotypes to crown gall disease may result from a deficiency in T-DNA integration. *Plant Cell* 8, 873-886 (1997).
Narasimhulu, S. B., Deng, X.-B. Sarria, R. & Gelvin, S. B. Early transcription of *Agrobacterium* T-DNA genes in tobacco and maize. *Plant Cell* 8, 873-886 (1996).
Ni, M. *et al*. Strength and tissue specificity of chimeric promoters derived from the octopine and mannopine synthase genes. Plant J. 7, 661-676 (1995).
Offringa, R. *et al*. Extrachromosomal homologous recombination and gene targeting in plant cells after *Agrobacterium* mediated transformation. *EMBO J. 9,* 3077-3084 (1990).
Ohba, T., Yoshioka, Y., Machida, C. & Machida, Y. DNA rearrangement associated with the integration of T-DNA in tobacco: An example for multiple duplications of DNA around the integration target. *Plant J.* 7, 157-164 (1995).
Paszkowski, J., Baur, M., Bogucki, A. & Potrykus, I. Gene targeting in plants. *EMBO J .* 7, 4021-4026 (1988).
Sambrook, M. A., Fritsch, E. F., & Maniatis, T. (1982) in *Molecular cloning: A laboratory manual*. 1^{st} ed. (Cold Spring Harbor Laboratory Press, Cold Spring Harbor, New York)
Sheng, J. & Citovsky, V. *Agrobacterium-plant* cell DNA transport: Have virulence proteins, will travel. *Plant Cell* 8,1699-1710 (1996).
Tao, Y., Rao, P. & Gelvin, S. B. A plant phosphatase is involved in nuclear import of the *Agrobacterium* VirD2/T-DNA complex. In preparation.
Zupan, J. R. & Zambryski, P. The *Agrobacterium* DNA transfer complex. *Crit. Rev. Plant Sci.* 16, 279-295 (1997).

**Table 1.**

| Complementation of the *rat5* mutant and overexpression of *RAT5* in wild-type (Ws) *Arabidopsis* plants | | |
|---|---|---|
| % Line^{a} | Root Bundles With Tumors | Tumor morphology |
| *rat5* complementation with At1012 (T2 plants)^{a} | | |
| Ws | 98±2 | large, green |
| *rat5* | 21±6 | small, yellow |
| *rat5* At1012-1 | 64±30 | large + small, green |
| *rat5* At1012-2 | 17±4 | small, yellow |
| *rat5* At1012-3 | 70±20 | large + medium, green |
| *rat5* At 1012-4 | 86±6 | large, green |
| *rat5* At1012-5 | 82±10 | large, green |
| *rat5* At1012-6 | 92±5 | large, green |

| Overexpression of *RAT5* in Ws (T2 plants) ^{a}^{b} | | |
|---|---|---|
| Ws | 35±14 | large, green |
| Ws At1012-1 | 69±27 | large, green |
| Ws At1012-2 | 68±25 | large, green |
| Ws At1012-3 | 64±13 | large, green |
| Ws At1012-4 | 63±20 | large, green |

| | | |
|---|---|---|
| ^{a}at least 5 plants were tested for each mutant and 40-50 root bundles were tested for each plant | | |
| ^{b}*Agrobacterium* was diluted to a concentration 100-fold lower than that normally used, and single root segments were separated | | |

## Claims

1. A method of increasing transformation frequencies in a host plant, said method comprising:
a. adding at least one copy of a plant histone gene to the host plant; and
b. expressing the copies of the plant histone gene to increase transformation frequencies.

2. The method of claim, 1 wherein the histone gene is an H2A gene.

3. A transgenic plant comprising at least one additional copy of a histone gene for increasing transformation frequencies in a host plant by overexpressing histone as compound to the host's natural expression levels.

4. The transgenic plant of claim 3, wherein the histone gene is the *RAT5* gene of *Arabidopsis*..

5. Use of a genetic construct comprising at least one copy of a histone gene for increasing transformation frequencies in a host plant by overexpressing histone as compound to the host's natural expression levels.

6. The use of claim 5, wherein the histone gene is H2A.

7. The use of claim 6, wherein the histone gene is a *RAT5 Arabidopsis* gene.

## Patentansprüche

1. Verfahren zum Erhöhen von Transformationshäufigkeiten in einer Wirtspflanze, wobei das Verfahren das
a. Hinzufügen von mindestens einer Kopie eines Pflanzen-Histon-Gens zu der Wirtspflanze; und
b. Exprimieren der Kopien des Pflanzen-Histon-Gens zur Erhöhung der Transformationshäufigkeiten
umfasst.

2. Verfahren nach Anspruch 1, wobei das Histon-Gen ein H2A-Gen ist.

3. Transgene Pflanze, die mindestens eine zusätzliche Kopie eines Histon-Gens zum Erhöhen von Transformationshäufigkeiten in einer Wirtspflanze durch Überexprimieren von Histon als Zusammensetzung zu den natürlichen Expressionsspiegeln des Wirtes umfasst.

4. Transgene Pflanze nach Anspruch 3, wobei das Histon-Gen das *RAT5*-Gen von *Arabidopsis* ist.

5. Verwendung eines genetischen Konstrukts, das mindestens eine Kopie eines Histon-Gens zum Erhöhen von Transformationshäufigkeiten in einer Wirtspflanze durch Überexprimieren von Histon als Zusammensetzung zu den natürlichen Expressionsspiegeln des Wirtes umfasst.

6. Verwendung nach Anspruch 5, wobei das Histon-Gen H2A ist.

7. Verwendung nach Anspruch 6, wobei das Histon-Gen ein *RAT5*-Gen von *Arabidopsis* ist.

## Revendications

1. Méthode pour augmenter les fréquences de transformation dans une plante-hôte, ladite méthode comprenant :
a) l'ajout d'au moins une copie d'un gène d'histone de plante à la plante-hôte ; et
b) l'expression des copies du gène d'histone de plante pour augmenter les fréquences de transformation.

2. Méthode de la revendication 1 dans laquelle le gène d'histone est un gène H2A.

3. Plante transgénique comprenant au moins une copie supplémentaire d'un gène d'histone pour augmenter les fréquences de transformation dans une plante-hôte en surexprimant l'histone en tant que composé à des niveaux d'expression naturels de l'hôte.

4. Plante transgénique de la revendication 3, dans laquelle le gène d'histone est le gène *RAT5* d'*Arabidopsis*.

5. Utilisation d'une construction génétique comprenant au moins une copie d'un gène d'histone pour augmenter les fréquences de transformation dans une plante-hôte en surexprimant l'histone en tant que composé à des niveaux d'expression naturels de l'hôte.

6. Utilisation de la revendication 5, dans laquelle le gène d'histone est H2A.

7. Utilisation de la revendication 6, dans laquelle le gène d'histone est un gène *RAT5* d'*Arabidopsis*.
